# EUROPEAN PATENT APPLICATION

(11) **EP 4 450 077 A1**
(43) Date of publication of application: **23.10.2024**
(21) Application number: 24170748.8
(22) Date of filing: 17.04.2024
(51) Int. Cl.: A61K 36/185, A61K 8/35, A61K 8/365, A61P 15/02

(54) **FORMULATIONS COMPRISING HENNA PLANT EXTRACTS, METHODS FOR THEIR PREPARATION AND USES THEREOF FOR TREATING FUNGAL INFECTIONS**

(30) Priority: 19.04.2023 US 202363497055 P
(71) Applicant: Aristotle University of Thessaloniki - E.L.K.E. (Eidikos Logariasmos Kondilion Erevnas), 54636 Thessaloniki (GR); Migal Galilee Research Institute Ltd., 11016 Kyriat Shmona (IL)
(72) Inventor: KOUMBIS, Alexandros, 54124 Thessaloniki (GR); ROILIDES, Emmanuel, 54642 Thessaloniki (GR); VAYA, Jacob, 13802 Merom Hagalilee (IL)
(74) Representative: Petsis, Christos

(57) **Abstract**

The invention relates to a formulation comprising an extract of *Lawsonia inermis,* wherein the extract is a 70% aqueous ethanol extract of *Lawsonia inermis,*
as well as to a method for the preparation of a formulation comprising a 70% aqueous ethanol extract of *Lawsonia inermis,* or polar fractions thereof, having enhanced anti-fungal activity, wherein the method comprises :
- dissolving powder of dried *Lawsonia inermis* leaves in a solvent which is 70% aqueous ethanol to obtain a suspension, and filtering said suspension to obtain a filtrate; and
- evaporating the solvent from the filtrate to obtain crude extract material.

## Description

### FIELD OF THE INVENTION

The present invention relates to formulations of extracts of *Lawsonia inermis* (henna tree) for the treatment of fungal diseases. More particularly, the invention relates to formulations comprising active fractions of *Lawsonia inermis* extracts for the treatment of fungal diseases, methods for their preparation, and uses thereof in anti-fungal treatment.

### BACKGROUND OF THE INVENTION

Fungal infections affecting the skin, hairs, nails, and mucous membranes with high prevalence are becoming a public health concern worldwide. Infections with *Candida albicans* are in particular associated with high morbidity and mortality. Immunocompromised patients, including patients receiving immunosuppressives, are even at higher risk of fungal infection. The presently common synthetic drugs (such as amphotericin B, Clotrimazole, and fluconazole) are prone to several drawbacks, including the development of resistance, gastrointestinal disturbances, skin rashes, arrhythmia, and hepatotoxicity.

*Lawsonia inermis* (henna) is a shrub native to subtropical and tropical countries. Lawsone (2-hydroxy-1,4-naphthoquinone), also known as hennotannic acid, is a red-orange dye present in the leaves of the henna plant and has been used for thousands of years as a dye for skin, hair, and fingernails, as well as fabrics. Extracts from the henna plant are also known to have medicinal properties, such as antibacterial, antifungal, antiparasitic, antioxidant, analgesic, anti-inflammatory, antipyretic, wound and burn healing, immunomodulatory, antidiabetic, hypolipidemic, antiulcer, diuretic, and anticancer activities. These beneficial activities are currently described for crude henna extracts and lawsone is referred to as the sole active agent in said crude extracts. However, the crude extract does not distinguish active materials from non-active materials and thus formulations comprising crude henna extracts also contain materials that are essentially devoid of anti-fungal activity and reduce the solubility of the extract in aqueous solutions, thereby rendering the formulation less effective.

It is therefore an object of the present invention to provide improved formulations of *Lawsonia inermis* extract that are enriched with fractions of said extract having enhanced anti-fungal activity.

It is another object of the invention to provide a method for the preparation of said improved formulations of *Lawsonia inermis* extract.

Other objects and advantages of the invention will become apparent as the description proceeds.

### SUMMARY OF THE INVENTION

In one aspect of the invention, there is provided a formulation comprising an extract of *Lawsonia inermis,* wherein the extract is a 70% aqueous ethanol extract of Lawsonia inermis.

In one embodiment of the invention, the formulation comprises the polar fraction of the 70% aqueous ethanol extract.

In another embodiment of the invention, the formulation comprises fractions of the 70% aqueous ethanol extract having middle and high polarity.

In a further embodiment of the invention, the formulation further comprises a detergent.

According to yet a further embodiment of the invention, the formulation further comprises an acid.

According to another embodiment of the invention, the formulation comprises compounds, which are:
- leucodelphinidin 3-glucoside;
- lawsoniaside; and
- 1,2,4-trihydroxynaphthalene-1-O-β-D-glucopyranoside,
either alone or in combination, optionally further comprising lawsone.

In another aspect of the invention, there is provided a formulation for use in treating fungal infections or a method of treating fungal infections, the method comprising administering the formulations described above.

In one embodiment of the invention, the formulation is administered orally, topically, or intravaginally.

In another embodiment of the invention, the formulation is in the form of a liquid, semi-solid, or solid dosage form.

In yet another embodiment of the invention, the formulation is applied to a feminine hygiene product.

In some embodiments of the invention, the fungal infection is selected from the group consisting of a systemic infection, a localized fungal infection, and a combination thereof.

In a further aspect of the invention, there is provided a method for the preparation of a formulation comprising a 70% aqueous ethanol extract of *Lawsonia inermis,* or polar fractions thereof, having enhanced anti-fungal activity, the method comprising:
- dissolving powder of dried *Lawsonia inermis* leaves in a solvent which is 70% aqueous ethanol to obtain a suspension, and filtering said suspension to obtain a filtrate; and
- evaporating the solvent from the filtrate to obtain crude extract material.

According to one embodiment of the invention, the method further comprises:
- adding a low-polarity solvent to the extract material to obtain a suspension, and filtering said suspension to obtain a filtrate and a residual; and
- drying the residual to obtain polarfraction material.

According to another embodiment of the invention, the method further comprises:
- isolating the fractions of the extract material or polar fraction material having middle and high polarity to obtain isolated polar fractions.

According to yet another embodiment of the invention, the method further comprises:
- diluting the crude extract material, the polar fraction material, or isolated polar fractions in water, optionally in the presence of a detergent.

In yet a further aspect of the invention, there is provided a method for increasing the solubility of Lawsonia Inermis extracts in water, the method comprising:
- preparing an extract material, a polar fraction material or isolated polar fractions of *Lawsonia inermis* according to the methods described herein; and
- dissolving said extract material, polar fraction material or isolated polar fractions in water in the presence of a detergent to obtain a solution.

In yet another aspect of the invention, there is provided a method for enhancing the anti-fungal activity of 70% aqueous ethanol extracts of *Lawsonia inermis,* or fractions thereof, the method comprising:
- preparing an extract material, a polar fraction material or isolated polar fractions of *Lawsonia inermis* according to the methods described herein; and
- dissolving said extract material, polar fraction material or isolated polar fractions in water, optionally in the presence of a detergent, to obtain a solution; and
- adding an acid to the solution and heating said solution.

According to one embodiment of the invention, the detergent is a non-ionic surfactant.

According to a specific embodiment of the invention, the acid is acetic acid.

### BRIEF DESCRIPTION OF THE DRAWINGS

**Fig**. **1** shows the anti-fungal activity of different concentrations of ethanol- and water-based extracts of *Lawsonia inermis* dissolved in water, presented as % reduction of the metabolic activity of *Candida albicans* in XTT assay. Abbreviations: anti-fungal activity (AF); ethanol (EtOH); 70% aqueous ethanol (EtOH-70); Water (W).
**Figs. 2A-2B** show the effects of the addition of acid to different concentrations of ethanol-based extracts of *Lawsonia inermis* on their anti-fungal activity presented as % reduction of the metabolic activity of *Candida albicans* in XTT assay.
   Fig. 2A shows the effects of acetic acid addition to different concentrations of a pure ethanol extract of *Lawsonia inermis* on anti-fungal activity.
   Fig. 2B shows the effects of acetic acid addition to different concentrations of a 70% aqueous ethanol extract of *Lawsonia inermis* on anti-fungal activity.
   Abbreviations: acetic acid (AcOH); anti-fungal activity (AF); ethanol (EtOH); 70% aqueous ethanol (EtOH-70).
**Fig. 3** shows the anti-fungal activity (AF) of different concentrations of the polar fraction of 70% aqueous ethanol extracts (after decantation of the crude extract by washing it with ethyl acetate), presented as % reduction of the metabolic activity of *Candida albicans* in XTT assay.
**Fig. 4** shows the anti-fungal activity (AF) of different concentrations of commercially available Lawsone, presented as % reduction of the metabolic activity of *Candida albicans* in XTT assay.
**Fig. 5** shows the anti-fungal activity, presented as % reduction of the metabolic activity of *Candida albicans* in XTT assay, of various concentrations of selected fractions separated from the polar fraction of the 70% aqueous ethanol extract. Abbreviations: anti-fungal activity (AF); polar fraction (PF).

### DETAILED DESCRIPTION OF THE INVENTION

The present invention provides an improved natural phytochemical product for the treatment of fungal diseases such as Candidiasis, as well as other mycotic infections. The formulations of the invention, comprising the polar fractions out of the crude extract of the henna plant, have enhanced anti-fungal activity over the crude extract or commercially available lawsone that are known from the prior art. This finding is important since the formulations provided here are enriched in compounds with higher polarity, having high anti-fungal activity and this in turn increases the efficacy of the formulations and allows the use of low concentrations of those high-activity extracts , thus reducing any side effects while maintaining the efficacy of the formulations compared to the extracts of the prior art. Another advantage of the formulations of the invention is its efficacy against species of fungi that are known to be resitant against known anti-fungal drugs, such as *Candida auris.*

In one aspect, the present invention provides a formulation comprising an extract of *Lawsonia inermis,* wherein the extract is a 70% aqueous ethanol extract of *Lawsonia inermis.*

In one embodiment of the invention, the concentration of the 70% ethanol crude extract in the formulation ranges between 9.5 µg/mL and 2500 µg/mL. In a specific embodiment of the invention, the formulation comprises between 19 µg/mL and 2500 µg/mL of said extract. In another specific embodiment of the invention, the concentration of the extract in the formulation is between 39 µg/mL and 80 µg/mL.

In another embodiment of the invention, the formulation comprises the polar fraction of the 70% aqueous ethanol extract of *Lawsonia inermis.* The term "polar fraction" as used herein refers to compounds that are present in the 70% aqueous ethanol extract of *Lawsonia inermis* and are insoluble in ethyl acetate.

In one embodiment of the invention, the polar fraction of the extract is obtained by treating the crude extract to remove the non-polar fraction (i.e., the non-polar compounds), for example, by mixing a solvent in which only non-polar compounds are dissolved and removing said solvent along with non-polar compounds dissolved therein. The remaining material is therefore the polar fraction of the extract. In a specific embodiment of the invention, said solvent that dissolves non-polar compounds is ethyl acetate, hexane, heptane, and/or diethyl ether.

In another embodiment of the invention, the concentration of the polar fraction of the *Lawsonia inermis* 70% ethanol extract in the formulation ranges between 9.5 µg/mL and 2500 µg/mL. In a specific embodiment of the invention, the formulation comprises between 9.5 µg/mL and 1250 µg/mL of said extract. In another specific embodiment of the invention, the concentration of the extract in the formulation is between 39 µg/mL and 1250 µg/mL.

In a further embodiment of the invention, the formulation comprises the fractions of the 70% aqueous ethanol extract of *Lawsonia inermis* having middle to high polarity, namely fractions having polarity of at least 0.3 relative polarity. In a non-limiting example, the middleto high polarity fraction are obtained by column chromatography of the 70% aqueous ethanol extract of *Lawsonia inermis* (after washing with ethyl acetate) on silica gel, eluting with increasingly polar solvents.

According to one embodiment of the invention, the formulation comprises the fraction of the extract having middle or high polarity at a concenrtaion ranging between 9.5 µg/ml and 2500 µg/ml. According to a specific embodiment of the invention, the concentration of the middle and high polarity fractions in the formulation is 9.5 to 1250 µg/ml. According to another specific embodiment of the invention, the concentration of the middle and high polarity fractions in the formulation is 9.5 to 50 µg/ml. According to yet another specific embodiment of the invention, the concentration of the middle and high polarity fractions in the formulation is 9.5 to 35 µg/mL.

The term "fraction" as used herein refers to a portion of the extract having a specific characteristic distinguishing one fraction from another. In the context of the present invention, the distinguishing characteristic is the polarity of the fractions, such that the crude extract generally comprises two main fractions: (1) non-polar fraction (also termed herein oily fraction), and (2) polar fraction. The polar fraction is further subdivided into: (a) low polarity fraction, (b) middle polarity fraction, and (c) high polarity fraction. It should be noted that each of the above-defined fractions consists of a single compound or a combination of compounds.

In some embodiments of the invention, the formulation further comprises one or more pharmaceutically acceptable excipients or carriers. The pharmaceutical excipients or carriers are selected from the group consisting of polymers, diluents, surfactants, emulsifiers, and the like.

In another embodiment of the invention, the formulations are in the form of liquid, semi-solid, or solid dosage forms. Accordingly, the formulations may take the form of tablets, pills, capsules, cachets, lozenges, granules, semisolids, powders, immediate- or sustained-release formulations, solutions, suspensions, emulsions, elixirs, aerosols, cream, ointment, paste, or any other appropriate dosage form.

In a further embodiment of the invention, the formulation comprises a detergent containing a polyethoxyethanol residue. In some embodiments of the invention, the detergent is a non-ionic, anionic, or zwitterionic surfactant. According to a specific embodiment of the invention, the detergent is a non-ionic surfactant. In another specific embodiment of the invention, the non-ionic surfactant is Polysorbate 20 (e.g., Tween^{®}-20). In a further specific embodiment of the invention, the detergent is present in the formulation at a concentration of 1-5% (v/v). According to yet another specific embodiment of the invention, the detergent is present in the formulation at a concentration of 2% (v/v).

In yet another embodiment of the invention, the formulation further comprises an acid. According to a specific embodiment of the invention, the acid is a carboxylic acid (such as acetic acid, formic acid, and trifluoroacetic acid). According to a further specific embodiment of the invention, the acid is acetic acid. According to one embodiment of the invention, the acid is present in the formulation at a concentration of 0.5-5% (v/v). In a specific embodiment of the invention, the acid is present in the formulation at a concentration of 1% (v/v).

According to a specific embodiment of the invention, the concentration of the acid-treated polar fraction of the *Lawsonia inermis* 70% ethanol extract in the formulation ranges between 9.5 µg/mL and 2500 µg/mL. In another specific embodiment of the invention, the formulation comprises between 19 µg/mL and 2500 µg/mL of said extract. In yet another specific embodiment of the invention, the concentration of the extract in the formulation is between 19 µg/mL and 40 µg/mL.

According to some embodiments, the formulation according to the present invention comprises the following compounds, either alone or in combination:
- leucodelphinidin 3-glucoside;
- lawsoniaside; and
- 1,2,4-trihydroxynaphthalene-1-*O*-*β*-D-glucopyranoside.

According to another embodiment of the invention, the formulation further comprises lawsone.

According to one embodiment of the invention, the above-named compounds are extracted from any plant material naturally comprising said compounds. In another embodiment of the invention, the compounds are purchased as commercially available products. According to a further embodiment of the invention, the compounds are chemically synthesized.

In another aspect of the invention, there is provided a method of treating a fungal infection/disease in a subject.

Accordingly, the formulations of the invention are used as anti-fungal agents, namely, for treating fungal infections/diseases.

In one embodiment of the invention, the subject is human or animal.

As used herein, the term "treatment" or "treating" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, a treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

As used herein, the term "prevent" or "preventing" refers to precluding, averting, obviating, forestalling, stopping, or hindering a disease, disorder, or pathological condition from happening, especially by advance action.

In some embodiments of the invention, the formulation is administered therapeutically; that is, administered to treat an existing disease, disorder, or condition. In other embodiments of the invention, the formulation is administered prophylactically; that is, administered for the prevention of a disease, disorder, or condition.

In one embodiment of the invention, the formulations are administered concurrently with, but not limited to, one or more additional anti-fungal agents. According to one embodiment of the invention, the one or more additional anti-fungal agents is selected from polyene anti-fungal drugs (such as amphotericin, nystatin, and pimaricin), azole anti-fungal drugs (such as fluconazole, clotrimazole, corbimazole, ravuconazole, itraconazole, voriconazole, posaconazole, isavuconazole, and ketoconazole), allylamine and morpholine anti-fungal drugs (such as naftifine and terbinafine), echinocandins (e.g., caspofungin, micafungin, and anidulafungin), and anti-metabolite anti-fungal drugs (such as 5-fluorocytosine).

As mentioned above, the term "treatment" encompasses the relief of symptoms of a disease. Accordingly, the present invention also encompasses a method for alleviating symptoms of a disease, disorder, or condition caused by a fungal infection by administering the formulations of the invention. In other words, the formulations of the invention are useful for alleviating symptoms of fungal diseases.

The term "symptom(s)" as used herein, refers to common signs or indications that a subject is suffering from a specific condition or disease. For example, symptoms associated with fungal infections include, but are not limited to fatigue, headache, muscle aches, night sweats, weight loss, chills, itch or soreness, scaly skin, redness, swelling, and fever.

Accordingly, the formulations of the invention are effective in reducing the severity and/or duration of the symptoms of diseases caused by a fungal infection, in enhancing the recovery of patients from the infection, and in reducing long-term morbidity, sequelae, or complications that result from the fungal infection.

The treatment and/or prevention of fungal infections and their symptoms may involve the manifestation of anti-fungal effects exhibited by formulations of the invention. These anti-fungal effects may include reduction of the pathogenicity of the fungus, inhibition of the fungus`s enzymes, (mainly proteases, phospholipases, and cytochrome P450-dependent enzymes such as C14-demethylase), inhibition of amino acid synthesis of the fungus, destruction of the fungal cell membrane and function, and inhibition of both DNA and RNA synthesis.

The fungal infection to be treated (or prevented) by the formulations of the present invention is selected from the group consisting of a systemic infection, a localized fungal infection, and a combination thereof.

The fungal diseases/infections that can be treated by the formulations of the invention comprise, but are not limited to, Candidiasis (caused, for example, by *C*. *albicans, C. Auris, C. dubliniensis, C. tropicalis, C. glabrata,* and *C. parapsilosis*), Aspergillosis, Blastomycosis, Valley Fever (Coccidioidomycosis), Cryptcoccosis and other Cryptococcus infections (caused, for example, by *C. neoformans* and *C. gattii*), fungal nail infections, Mucormycosis, Mycetoma, Dermatophytosis (also known as Tinea or Ringworm), Sporotrichosis (also known as Rose Gardener's Disease), Paracoccidioidomycosis, Talaromycosis, Chromoblastomycosis, Emergomycosis, Fusariosis, and Scedosporiosis.

Examples of the fungal species that the formulations of the invention are useful against include, but are not limited to, Candida spp (such as *C*. *albicans, C. Auris, C. dubliniensis, C. tropicalis, C. glabrata,* and *C*. *parapsilosis*)*, Acrophialophora fusispora, Aspergillus flavus, Aspergillus niger, Cladosporium spp* (such as *Cladosporium herbarum*)*, Emerisella spp, Epidermophton floccosum, Fusarium* sp., *Malassezia* sp., *Microsporum audoiinii, Microsporum canis, Microsporum fulvum, Microsporum gypseum, Penicillium* sp., *Rhizopus spp* (such as *Rhizopus stolinifer*)*, Syncephalastrum racemosum, Trichoderma* sp., *Trichophyton concentricum, Trichophyton mentagrophytes, Trichophyton rubrum, Trichophyton tonsurans, Trichophyton verrucosum,* and *Trichophyton violaceum.*

In general, the formulations of the invention will be administered as pharmaceutical compositions by one of the following routes: oral, topical, otic, ocular, vaginal, systemic (e.g. transdermal, intranasal, intrapulmonary or by suppository), parenteral (e.g. intramuscular, subcutaneous, intra-arterial, intraperitoneal or intravenous injection), by implantation and by infusion through such devices as osmotic pumps, transdermal patches and the like.

According to one embodiment of the invention, the formulations described herein are applied to feminine hygiene products (such as pads and wipes), and female and male contraceptive devices.

According to another embodiment of the invention, the formulations are administered in the form of a suppository, toothpaste, mouthwash, and vaginal lubricant.

Accordingto a further embodiment of the invention, the formulations are in the form of dietary supplements or nutritional supplements. As used herein, the term "dietary supplement" refers to a small amount of a compound, composition, or combination of compounds for supplementation of a human or animal diet, wherein the dietary supplement is packaged in single or multiple dose units. Dietary supplements do not provide significant amounts of calories but may contain other micronutrients (e.g., vitamins or minerals). The dietary supplements according to the present invention may be administered in any form (e.g., solution, pill, powder, or food product). The dietary supplements may further comprise one or more inert ingredients. For example, the dietary supplements may also contain vitamins, minerals, enhancers, colorants, sweeteners, flavorants, preservatives, and/or inert ingredients.

In one embodiment of the invention, the dietary supplements are provided in liquid form to be used by adding to a drink (e.g., water, milk, juice, a carbonated drink, a hot drink, or liquor), solid food (such as a slice of bread) or semi-solid food (such as a pudding, topping, sauce, puree, cooked cereal, or salad dressing.

The term "nutritional supplement" as used herein refers to a combination of a dietary supplement and a source of calories. In some embodiments of the invention, nutritional supplements are meal replacements or meal supplements (such as a nutrient or energy bar or a nutrient beverage, shake, or concentrate).

In a further aspect, the present invention provides a method for the preparation of a formulation comprising a 70% aqueous ethanol extract of *Lawsonia inermis,* or polar fractions thereof, having enhanced anti-fungal activity as described herein above, the method comprising:
- dissolving powder of dried *Lawsonia inermis* leaves in a solvent which is 70% aqueous ethanol to obtain a suspension, and filtering said suspension to obtain a filtrate; and
- evaporating the solvent from the filtrate to obtain extract material.

As can be appreciated by a person of skills in the art, a powder of dried henna leaves can be obtained using standard techniques. For example, plucking leaves of the henna plant, optionally washing the leaves, leaving the leaves to dry completely (preferably in the shade), grinding or crushing the leaves into a powder, and optionally straining the powder through a sieve or muslin cloth to obtain a fine powder.

The terms "dissolve" and "dissolving" as used herein refer to causing a material, for example, a powder, to become incorporated into a liquid so as to form a solution or a suspension. The terms encompass utilizing standard actions, such as stirring (manually or using a stirrer), heating, cooling, incubating, and the like, for a predetermined period of time, or until the material is entirely incorporated into the liquid.

The terms "70% aqueous ethanol" and "70% ethanol" as used interchangeably herein refer to a mixture of ethanol and water, wherein the ethanol makes 70% (v/v) of the mixture.

As would be appreciated by a person of skills in the art, during the step of dissolving powder of dried *Lawsonia inermis* leaves in a solvent in the above-described method there may be some elements in the leaves powder that are not dissolved and remain solid in the solution.

Filtration of the solution is carried out to remove parts of henna leaves and elements that were not completely dissolved in the solvent. The filtration can be carried out using well-known filtration techniques, such as through filter paper, with or without the aid of celite^{®}.

Evaporation of solvents is carried out using routine techniques, such as rotary evaporation. The evaporation usually continues until the material is substantially or entirely depleted of liquid solvent. Accordingly, the extract material obtained after evaporation is essentially in solid form, i.e., dry powder.

In some embodiments of the invention, the above-defined dissolving and evaporating steps of the method can be carried out once. In other embodiments of the invention, the dissolving and evaporating cycle can be repeated one or more times.

In one embodiment of the invention, the method further comprises:
- adding a low polarity solvent to the extract material to obtain a suspension, and filtering said suspension to obtain a filtrate which is the oily fraction and a residual which is the polar fraction; and
- drying the residual to obtain polar fraction material.

According to an embodiment of the invention, the non-polar solvent is selected from ethyl acetate, alkanes (such as pentane, hexane, and heptane), diethyl ether, aromatics (such as toluene, benzene, and xylene), and the like. In a specific embodiment of the invention, the non-polar solvent is ethyl acetate.

The term "low-polarity solvent" as used herein refers to a solvent having a polarity of less than 0.3 relative polarity. Of course, solvents known in the art as non-polar are also encompassed by the term "low-polarity solvent".

The drying of the residual is carried out by routine methods. According to one embodiment of the invention, the drying of the residual is carried out under vacuum. Accordingly, the polar fraction material obtained after drying is essentially in solid form, i.e., dry powder.

It should be noted that the filtrate may be evaporated so as to obtain oily fraction material.

In some embodiments of the invention, the above-defined adding and drying steps of the method can be carried out once. In other embodiments of the invention, the adding and drying cycle can be repeated one or more times.

Alternatively, the polar fraction of the crude extract can be isolated from the crude extract using standard separation and isolation techniques, such as chromatography (e.g., column chromatography, High-performance liquid chromatography (HPLC), preparative HPLC, open column flash chromatography, etc.), and liquid-liquid extraction.

According to another embodiment of the invention, the method further comprises isolating the fractions of the extract having middle and high polarity to obtain isolated polar fractions.

As mentioned above, an isolated polar fraction may consist of a single compound or a mixture of compounds.

The polarity of each fraction is determined by the polarity of the solvent that elutes said fraction from a chromatographic column. Accordingly, the fractions having middle polarity are those that are eluted from the column using the solvents: acetone, 2-propanol, 2-butanol, ethyl acetoacetate, and other solvents having similar polarity, or any combination of solvents resulting in a similar polarity. The fractions having high polarity are those that are eluted from the column using the solvents: ethanol, methanol, ethylene glycol, water, formic acid, and other solvents having similar polarity, or any combination of solvents resulting in a similar polarity.

According to a specific embodiment of the invention, the method further comprises isolating the following compounds of the henna extract:
- leucodelphinidin 3-glucoside;
- lawsoniaside; and
- 1,2,4-trihydroxynaphthalene-1-*O*-*β*-D-glucopyranoside; and optionally
- lawsone.

Isolation of specific fractions or compounds from the crude extract or certain fractions of the extract is carried out using standard separation and isolation techniques, such as chromatography (e.g., column chromatography, High- performance liquid chromatography (HPLC), preparative HPLC, open column flash chromatography, etc.) and liquid-liquid extraction.

In general, the organic solvents of each fraction obtained, are evaporated to obtain the fraction in a solid form. In one embodiment of the invention, the different forms can be dissolved in a suitable solvent or diluted in a suitable diluent, optionally with one or more excipients or carriers, to form the formulations of the invention. According to a specific embodiment of the invention, the solvent/diluent is water. Thus, according to a further embodiment of the invention, the method for the preparation of the formulations of the invention further comprises dissolving/diluting the crude extract material, the polar fraction material, or isolated polar fractions or compounds in water, optionally in the presence of a detergent containing a polyethoxyethanol residue, particularly a non-ionic surfactant.

In yet another aspect, the present invention provides a method for increasing the solubility of henna plant extracts, or fractions thereof, in water, the method comprising:
- preparing an extract material, polar fraction material or isolated fractions of *Lawsonia inermis* according to the methods of the invention; and
- dissolving said extract material, polar fraction material or isolated fractions in water in the presence of a detergent to obtain a solution.

In some embodiments of the invention, the detergent contains a polyethoxyethanol residue. According to one embodiment of the invention, the detergent is a non-ionic surfactant. According to another embodiment of the invention, the non-ionic surfactant is Polysorbate-20 (Tween^{®}-20). According to another embodiment of the invention, the final concentration of the detergent in the solution is 1-5% (v/v). According to a specific embodiment of the invention, the detergent is present in the solution at a concentration of 2% (v/v).

In a further aspect, the present invention provides a method for enhancing the anti-fungal activity of 70% aqueous ethanol extracts of *Lawsonia inermis,* or fractions thereof, the method comprising:
- preparing an extract material, polar fraction material, or isolated fractions of *Lawsonia inermis* according to the methods of the invention;
- dissolving said extract material, polar fraction material, or isolated fractions in water, optionally in the presence of a detergent, to obtain a solution; and
- adding an acid to the solution and heating said solution.

In some embodiments of the invention, the detergent contains a polyethoxyethanol residue. According to one embodiment of the invention, the detergent is a non-ionic surfactant. According to another embodiment of the invention, the non-ionic surfactant is Polysorbate-20 (Tween^{®}-20). According to another embodiment of the invention, the final concentration of the detergent in the solution is 1-5% (v/v). According to a specific embodiment of the invention, the detergent is present in the solution at a concentration of 2% (v/v).

According to one embodiment of the invention, the acid is carboxylic acid (such as acetic acid, formic acid, and trifluoroacetic acid). According to a specific embodiment of the invention, the acid is acetic acid. According to another embodiment of the invention, the acid is present in the solution at a concentration of 0.5-5% (v/v). According to another specific embodiment of the invention, the final concentration of the acid in the solution is 1% (v/v).

In one embodiment of the invention the heating of the solution is carried out at 50-80 °C for a duration of 0.5-1.5 hours.

The invention will now be described with reference to specific examples and materials. The following examples are representative of techniques employed by the inventors in carrying out aspects of the present invention. It should be appreciated that while these techniques are exemplary of specific embodiments for the practice of the invention, those of skill in the art, in light of the present disclosure, will recognize that numerous modifications can be made without departing from the spirit and intended scope of the invention.

### EXAMPLES

### Materials and methods

### Preparation of Lawsonia inermis extracts

Extracts of the henna plant were prepared from dried leaves crushed or ground into a fine powder. 100 mL of solvent was added to 20 g of *Lawsonia Inermis* dried leaves powder and the mixture was stirred at room temperature for 1 hour and then filtered through celite^{®}. A second extraction was carried out on the same leaves that remained after filtration using 80 mL of the same types of solvent for an additional 1 hour and filtered through celite^{®}. The combined extracts were evaporated on a rotary evaporator under vacuum keeping the bath temperature at 45 °C to obtain extract material.

### Diluting Lawsonia Inermis extracts in water

In order to test the antifungal properties of the extracts, samples of each extract must be further dissolved in water. However, when using water as solvent or when trying to further dissolve the ethanol- and ethyl acetate-based extracts in water, the inventors found that none of the extracts properly dissolves in water. In order to overcome this obstacle, the extracts were dissolved in water containing a non-ionic surfactant, such as Polysorbate 20 (Tween^{®}-20), at a final concentration of 2% (v/v). In particular, a stock solution of 10 mg/mL or 5 mg/mL (equivalent to 10,000 and 5,000 ppm, respectively) was prepared by dissolving 10 mg or 5 mg, respectively, of each of the crude extracts in 40 µL of a mixture of TWEEN^{®}-20 and water at a ratio of 1:1 (v/v), then, 60 µL of water were added and the solution was mixed. The volume was then completed to 1 mL with water (900 µL) and the solution was further mixed.

### Acid treatment of extracts

Crude ethanol-based extracts prepared as described above were treated with acid such that an acid was added to the solution of water and surfactant as described above at a final concentration of 1% (v/v) and then heated. In particular, in a stock solution of 10 mg/mL, after dissolving 10 mg of the crude ethanol-based extracts in 40 µL of a mixture of TWEEN^{®}-20 and water at a ratio of 1:1 (v/v), 60 µL of a mixture of acetic acid and water at a ratio of 1:5 (v/v) was added and the solution was mixed. Then, the solution was heated for 1 hour at 80 °C and the volume was then completed to 1 mL with water (900 µL) and further mixed.

### Removal of oily fractions from Lawsonia inermis crude extract

*Lawsonia inermis* crude extract obtained using 70% aqueous ethanol as solvent was prepared by the methods described above. Ethyl acetate (200 mL) was added to the crude extract (6 g) and the solution was stirred for 30 minutes and then filtered, such that the filtrate contained the oily fraction and the residual contained the polar fraction. Afterwards, the solvent was evaporated from the filtrate.

### Isolation of components from the crude polar extract

A polar fraction of the 70% aqueous ethanol extract of *Lawsonia inermis* was prepared as mentioned above and subjected to separation by column chromatography as follows: 1 g of the polar extract was dissolved in 70% ethanol and mixed with Celite^{®} and the solvent was evaporated. The remaining powder was eluted on a silica gel column (10 cm). The following eluents were used: (1) ethyl acetate (EtOAc) (100 mL), (2) EtOAc:methanol (MeOH) 2% (containing 1% water) (100 mL), (3) 5% MeOH in ethyl acetate (200 mL), (4) 10% MeOH in ethyl acetate (300 mL), (5) 15% MeOH in ethyl acetate (200 mL), (6) 10% MeOH in acetone (200 mL), and (7) acetone:MeOH:formic acid (FA) 6:1:1. Based on thin-layer chromatography (TLC) visualization of spots, the fractions collected from the column chromatography were combined into the following 5 main fractions:
(A) F1 = a combination of fractions 1-2, 25.6 mg collected from EtOAc elution;
(B) F2 = a combination of fractions 3-5, 186.2 mg collected from 5% MeOH in ethyl acetate elution;
(C) F3 = a combination of fractions 6-9, 182.2 mg collected from 10% MeOH in ethyl acetate elution;
(D) F4 = a combination of fractions 10-13, 312 mg collected from 10% MeOH in acetone elution; and
(E) F5 = a combination of fractions 14-end, 332 mg collected from acetone:MeOH:FA 6:1:1.

It should be noted that under similar chromatography settings, lawsone and compounds having a similar retention factor (Rf), are optimally separated using dichloromethane (DCM):EtOAc:FA 5:1:0.1 as eluent. Optimal separation on TLC of peaks below Lawsone Rf is achieved using CHCl₃:EtOAc:FA 3:6:1 as eluent.

The F2 fraction was further chromatographed using the following eluents: DCM:EtOAc:FA (200 mL:40 mL:2 mL) and the following fractions were collected:
(A) F2A = 7.3 mg, in which lawsone was identified as the major component;
(B) F2B = 44.5 mg, pure one spot on TLC;
(C) F2C = 35 mg, a mixture of two spots on TLC; and
(D) F2D = 34.1 mg, pure one spot on TLC.

Fraction F2C was chromatographed again using the following solvents: CHCl₃:EtOAc:FA (3:6:0.5) 100 mL and then CHCl₃:EtOAc:FA 3:6:1 (300 mL). As fraction F2C seems to comprise a mixture of the compound of the F2B fraction and an additional compound with a lower Rf, the F2C fraction was chromatographed for a third time to isolate the compound with the lower Rf, which was labeled F2C1 (21.3mg).

The F3 fraction was also further chromatographed on silica gel using the following eluents: DCM:acetone 1:1 (200 mL) and then DCM:acetone:FA 1:1:0.1 (300 mL). Two pure compounds were collected and labeled F3A (16.5 mg) and F3B (19.5 mg). The chromatography was continued using EtOAc:Acetone:FA 1:1:0.1 as eluent and an additional pure compound was collected and labeled F3C (53.5 mg).

### Identification of isolated compounds after fractionation

The molecular structure of the pure compounds isolated from the crude extract of *Lawsonia inermis* was identified based on common spectroscopic data collected from nuclear magnetic resonance (NMR) spectroscopy (proton and carbon), mass spectrometry (MS), ultraviolet-visible (UV) spectroscopy, and Fourier transform infrared (FTIR) spectroscopy.

### Evaluation of anti-fungal activity

Fungal cultures of *Candida albicans* were prepared by inoculating a 10 µL loopful of *C. albicans* (from a freshly inoculated Sabouraud glucose agar plate) in 20 mL of Yeast Nitrogen Broth (YNB) medium and incubating overnight on a shaker at 37 °C. Yeast cells were harvested and washed twice with 0.15 M phosphate-buffered saline (PBS) solution (pH 7.2; Ca²⁺ and Mg²⁺ free) and then resuspended in 10 mL of PBS, counted after serial dilutions using a haemocytometer, standardized at 10⁶ blastoconidia/mL and seeded immediately for anti-fungal evaluation using 2,3-bis[2- methoxy-4-nitro-5-sulfophenyl]2H-tetrazolium-5-carboxanilide (XTT) reduction assay.

Anti-fungal activity of *Lawsonia inermis* extracts was determined in triplicates at 48 hours after seeding in RPMI 1640 supplemented with MOPS medium using a commercially available XTT assay kit according to the manufacturer's instructions. The % reduction of metabolic activity was quantitated spectrophotometrically at 450 nm. The lowest fraction concentration resulting in ≥50% reduction in the metabolic activity compared to untreated cultures was considered as the minimal inhibitory concentration (MIC).

### Evaluation of the susceptibility of Candida species to Lawsonia inermis extracts

Fungal cultures of *C*. *albicans* (types VC001 and VC029) and *C*. *auris* (types 1917 and 1929) were prepared using standard techniques. The minimal inhibitory concentration (MIC) of *Lawsonia inermis* extracts was determined following the Clinical and Laboratory Standards Institute (CLSI) guidelines as set out in the M27-A standard for broth microdilution (BMD) anti-fungal susceptibility assay.

The growth of the cultures treated with the polar fraction of the 70% aqueous ethanol extract of *Lawsonia inermis* (prepared as mentioned above) was observed at 24 hours of incubation.

MIC values were determined visually as the lowest extract concentration of extract resulting in ≥50% inhibition of fungal growth below control (untreated) levels.

### Example 1

### Anti-fungal activity of extracts was enhanced when using 70% aqueous ethanol as solvent

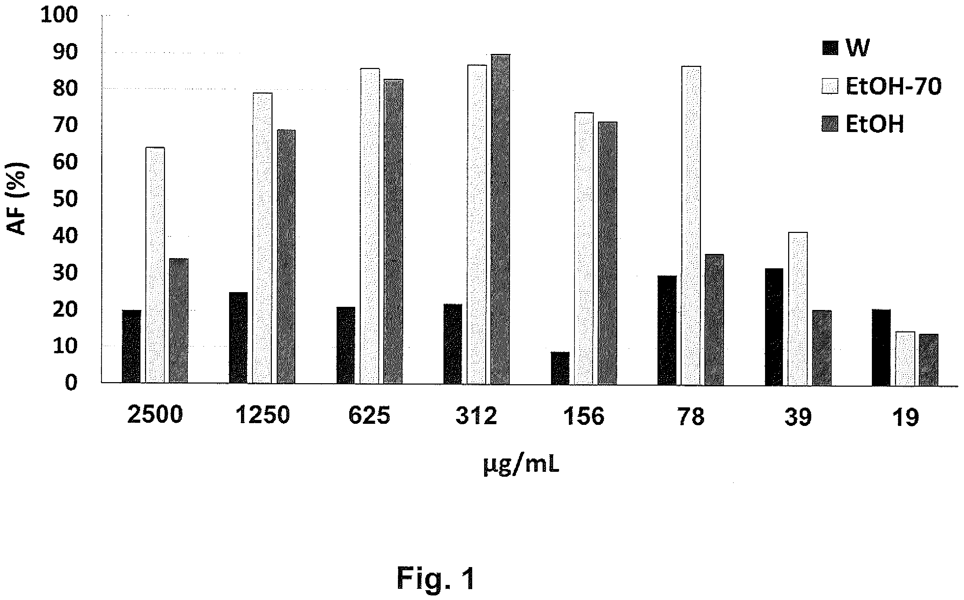

Anti-fungal activity was evaluated for four different types of *Lawsonia inermis* extracts using (1) water, (2) 70% aqueous ethanol, (3) ethanol, or (4) ethyl acetate as solvents. While the ethanol- and water-based extracts showed significant anti-fungal activity, the anti-fungal activity of the ethyl acetate extract was negligible. Fig. 1 shows the anti-fungal activity of the ethanol- and water-based extracts. The results indicate that the 70% aqueous ethanol extract of *Lawsonia inermis* had the highest activity at almost all concentrations tested.

### Example 2

### Acid treatment of the 70% ethanolic crude extract enhanced extract antifungal activity

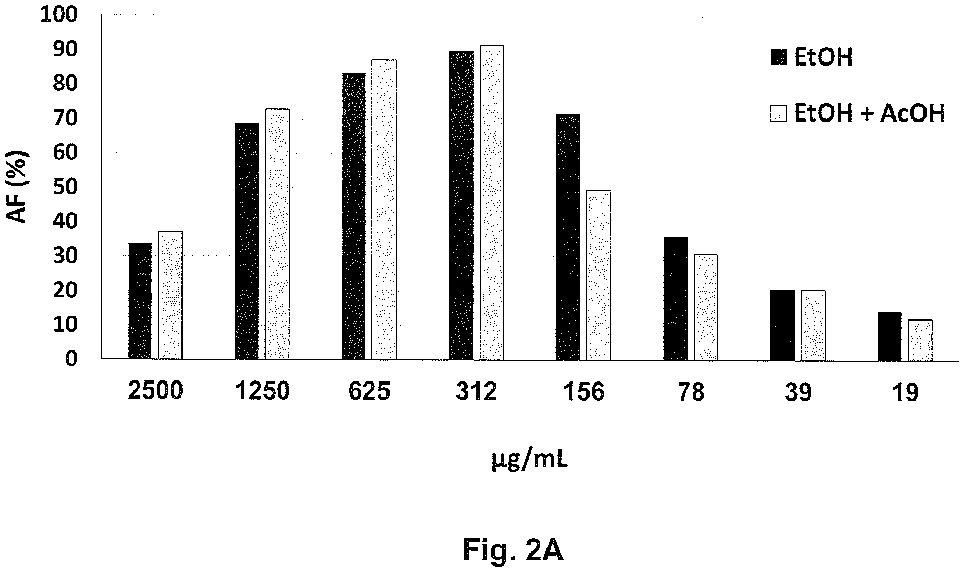

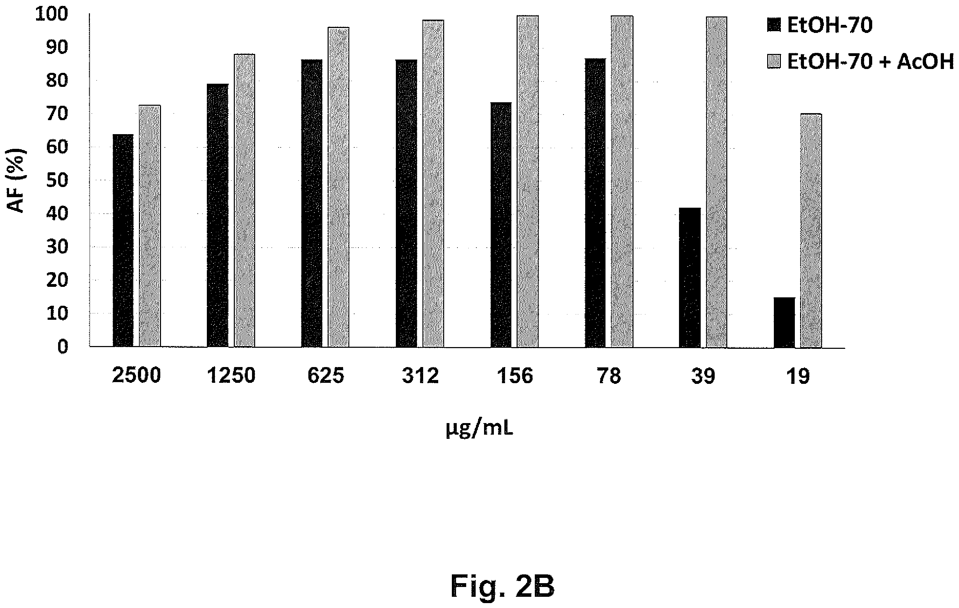

Acetic acid (AcOH) was added to the solution of pure ethanol extracts and of 70% aqueous ethanol extracts of the henna plant followed by heating. As shown in Fig. 2A, the acid treatment has no impact on the anti-fungal activity of the pure ethanol extract. However, Fig. 2B demonstrates that acid treatment using AcOH significantly improved the anti-fungal activity of the 70% aqueous ethanol extract.

### Example 3

### Enhanced anti-fungal activity of the polar fraction of 70% aqueous ethanol Lawsonia inermis extracts

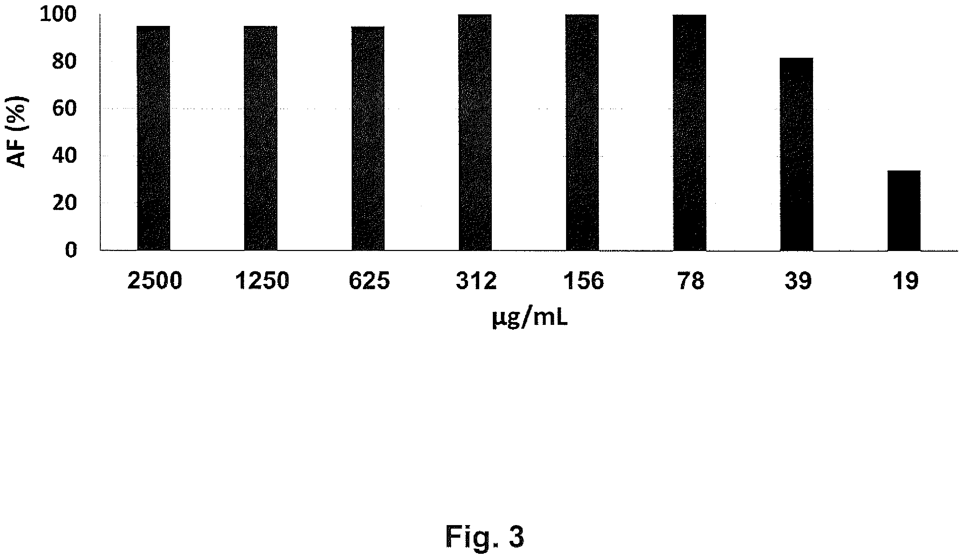

After establishing that the 70% aqueous ethanol extract of *Lawsonia inermis* is more efficient as an anti-fungal agent, the anti-fungal activity of the polar fraction of the extract was investigated. As shown in Fig. 3, the polar fraction of the 70% aqueous ethanol extract (the fraction that was insoluble in ethyl acetate) maintained the high anti-fungal activity of the *Lawsonia inermis* extract, indicating that the non-polar components of the extracts (namely, the components that were removed by washings with ethyl acetate) do not contribute to the anti-fungal activity of the extract.

### Example 4

### Isolation and identification of active compounds in Lawsonia inermis extract

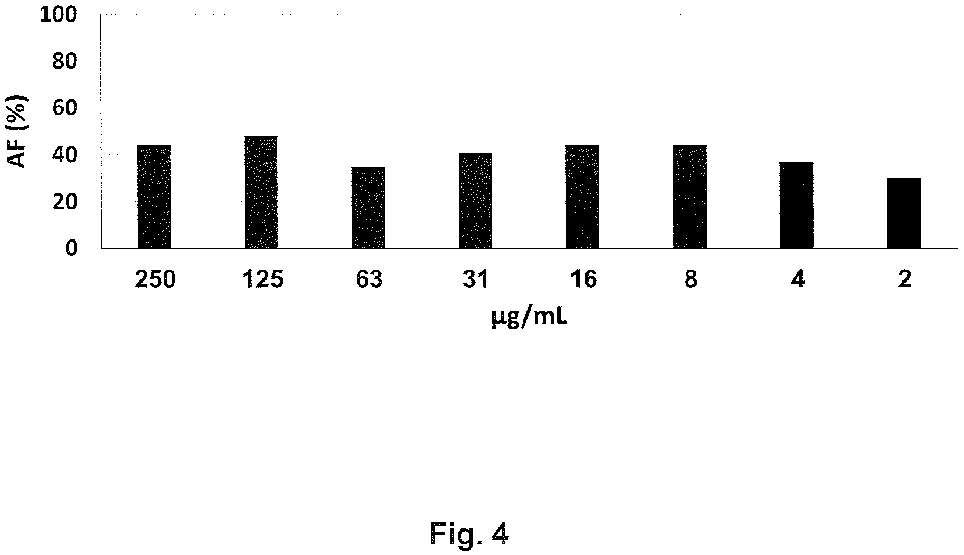

A comparison between the anti-fungal activity of the polar fraction of the 70% aqueous ethanol extract of *Lawsonia inermis* shown in Fig. 3 and the anti-fungal activity of commercially available lawsone shown in Fig. 4 indicates that lawsone is only partly responsible for the anti-fungal activity of the extract and that there are compounds in the polar fraction of the extract, other than lawsone, that contribute to the beneficial effects of the extract.

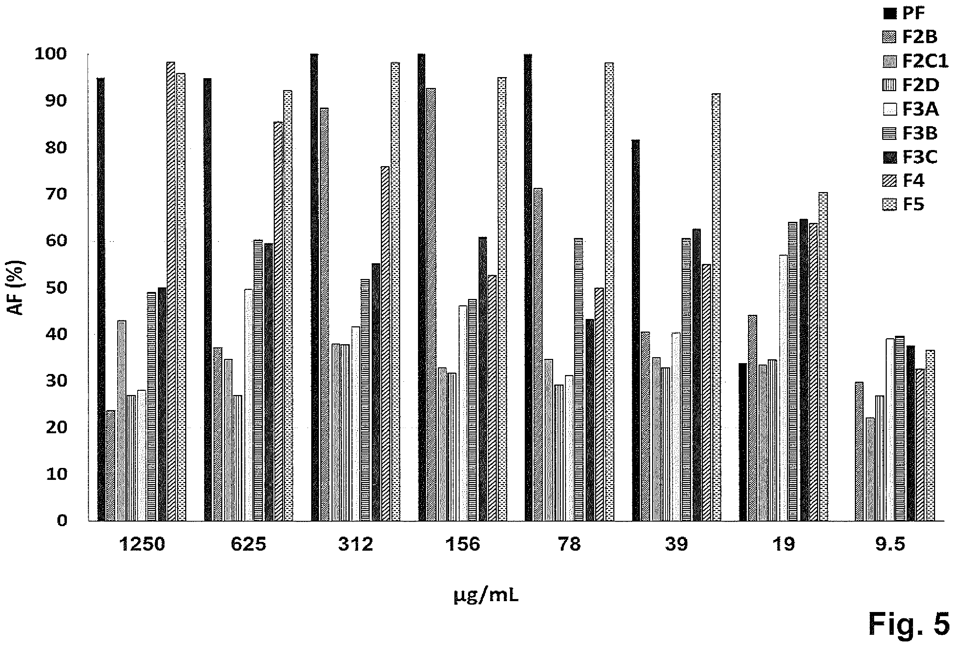

In light of the presence of active compounds other than lawsone in the polar fraction of the 70% ethanol extract of henna plant, a series of fractionations and separations was carried out by open column flash chromatography using silica gel and various solvents as eluents. Fractions F4 and F5 as defined herein above, as well as the pure compounds labeled F2B, F2C1, F2D, F3A, F3B, and F3C were tested for their anti-fungal activity compared to the efficiency of the entire polar fraction of the 70% aqueous ethanol extract. As shown in Fig. 5, compounds of middle polarity (such as F3A, F3B, and F3C) have moderate activity, while the most polar fractions (F4 and F5) exhibit the highest activity. Compounds of relatively lower polarity (isolated from the F2 fraction) are generally of lower activity. It should be also noted that the two semipurified fractions (F4 and F5) constitute more than 60% of the total extract, such that the anti-fungal activity of these fractions is contributing the major part of the antifungal activity of the crude extract.

As noted above, the single compound of fraction F2A was identified as lawsone (also known as 2-hydroxy-1,4-naphthoquinone or hennotannic acid), having the following structure:

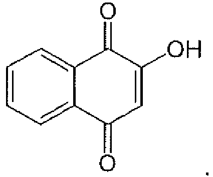

Standard identification techniques revealed that the single compound present in fraction F2B is 1,2,4-trihydroxynaphthalene-1-*O*-*β*-D-glucopyranoside (THNG) having the following structure:

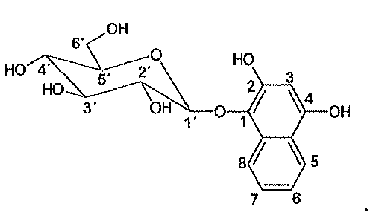

As would be appreciated by a person of skills in the art THNG is the glucosylated form of reduced lawsone. The conversion of THNG to Lawsone involves hydrolysis of THNG by β-glucosidases to an intermediate form (1,2,4-trihydroxynaphthalene) having the following chemical structure:

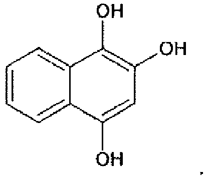

This intermediate form might be oxidized to finally yield lawsone.

The main compound in fraction F4 was identified as the compound of fraction F2B (THNG) having an additional glucose unit, namely lawsoniaside, also known as (2R,2'R,3S,3'S,4S,4'S,5R,5'R,6S,6'S)-6,6'-((2-hydroxynaphthalene-1,4-diyl)bis(oxy))bis(2-(hydroxymethyl)tetrahydro-2H-pyran-3,4,5-triol) having the following structure:

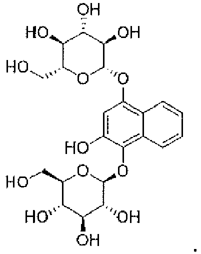

Fraction F5 contains a combination of two compounds. One of them is the same compound of fraction F4 and an additional compound that is not derived from lawsone subunit and is identified as leucodelphinidin 3-glucoside (IUPAC name: 3-[3,4,5-trihydroxy-6-(hydroxymethyl)oxan-2-yl]oxy-2-(3,4,5-trihydroxyphenyl)-3,4-dihydro-2H-chromene-4,5,7-triol] having the following structure:

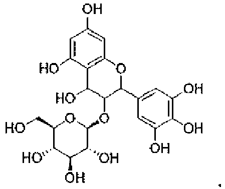

### Example 5

### Polar fractions of 70% aqueous ethanol Lawsonia inermis extracts are active against fungal species known to be resistant

Using a standard assay designed to determine the sensitivity of the various types of fungi to anti-fungal agents, the minimal inhibitory concentration (MIC) was determined for the polar fraction of 70% aqueous ethanol extracts of *Lawsonia inermis* grown in either Israel or Indonesia against two types of *C*. *albicans* (VC001 and VC029) and two types of *C*. *auris* (1917 and 1929). The MICs are shown in Table 1 below. The results indicate that the polar fraction of the 70% ethanol extract of the henna plant is also active against species of Candida that are otherwise known to be resistant to anti-fungal treatment (*C*. *auris*) with similar efficacy compared to the anti-fungal activity of these extracts against susceptible Candida species (*C*. *albicans*).

**Table 1. MIC values at 24 hours after incubation of different types of Candida treated with the polar fraction of the 70% aqueous ethanol extract of Israeli and Indonesian Lawsonia inermis.**

| **Species** | **Type** | **Israeli henna** | **Indonesian henna** |
|---|---|---|---|
| C. auris | 1917 | 16 mg/L | 1 mg/L |
| | 1929 | 4 mg/L | 4 mg/L |
| C. albicans | VC001 | 16 mg/L | 4 mg/L |
| | VC029 | 16 mg/L | 4 mg/L |

## Claims

1. A formulation comprising an extract of *Lawsonia inermis,* wherein the extract is a 70% aqueous ethanol extract of *Lawsonia inermis.*

2. The formulation of claim 1, wherein the formulation comprises the polar fraction of the 70% aqueous ethanol extract.

3. The formulation of claim 2, wherein the formulation comprises fractions of the 70% aqueous ethanol extract having middle and high polarity.

4. The formulation of any one of claims 1-3, further comprising a detergent.

5. The formulation of any one of claims 1-5, further comprising an acid.

6. A formulation comprising compounds, which are:
- leucodelphinidin 3-glucoside;
- lawsoniaside; and
- 1,2,4-trihydroxynaphthalene-1-*O*-*β*-D-glucopyranoside, either alone or in combination.

7. The formulation of claim 6, further comprising lawsone.

8. The formulation of any one of claims 1-7 for use in treating fungal infections.

9. A method of treating fungal infections, comprising administering the formulation of any one of claims 1-7 to a subject.

10. The formulation for use according to claim 8 or the method of claim 9, wherein the formulation is administered orally, topically, or intravaginally.

11. The formulation for use or the method according to claim 10, wherein the formulation is in the form of a liquid, semi-solid, or solid dosage form.

12. The formulation for use according to claim 8 or the method of claim 9, wherein the formulation is applied to a feminine hygiene product.

13. The formulation for use according to claim 8 or the method of claim 9, wherein the fungal infection is selected from the group consisting of a systemic infection, a localized fungal infection, and a combination thereof.

14. A method for the preparation of a formulation comprising a 70% aqueous ethanol extract of *Lawsonia inermis,* or polar fractions thereof, having enhanced anti-fungal activity, the method comprising:
- dissolving powder of dried *Lawsonia inermis* leaves in a solvent which is 70% aqueous ethanol to obtain a suspension, and filtering said suspension to obtain a filtrate; and
- evaporating the solvent from the filtrate to obtain crude extract material.

15. The method of claim 14, further comprising:
- adding a low polarity solvent to the extract material to obtain a suspension, and filtering said suspension to obtain a filtrate and a residual; and
- drying the residual to obtain polar fraction material.

16. The method of claim 14 or 15, further comprising:
- isolating the fractions of the extract material or polar fraction material having middle and high polarity to obtain isolated polar fractions.

17. The method of any one of claims 14-16, further comprising:
- diluting the extract material, the polar fraction material, or isolated polar fractions in water, optionally in the presence of a detergent.

18. A method for increasing the solubility of *Lawsonia Inermis* extracts, or fractions thereof, in water, the method comprising:
- preparing an extract material, a polar fraction material or isolated polar fractions of *Lawsonia inermis* according to any one of claims 14-16; and
- dissolving said extract material, polar fraction material or isolated polar fractions in water in the presence of a detergent to obtain a solution.

19. A method for enhancing the anti-fungal activity of 70% aqueous ethanol extracts of *Lawsonia inermis,* or fractions thereof, the method comprising:
- preparing an extract material, a polar fraction material or isolated polar fractions of *Lawsonia inermis* according to any one of claims 14-16;
- dissolving said extract material, polar fraction material or isolated polar fractions in water, optionally in the presence of a detergent, to obtain a solution; and
- adding an acid to the solution and heating said solution.

20. The formulation of claim 4 or the method of any one of claims 17 to 19, wherein the detergent is a non-ionic surfactant.

21. The method of claim 19 or 20, wherein the acid is acetic acid.
